# EUROPEAN PATENT APPLICATION

(11) **EP 4 159 756 A1**
(43) Date of publication of application: **05.04.2023**
(21) Application number: 20938753.9
(22) Date of filing: 02.06.2020
(51) Int. Cl.: C07K 16/08, C12N 15/13, G01N 33/53, G01N 33/569, G01N 33/576, G01N 33/577

(54) **ANTI-RNA VIRUS PARTICLE ANTIBODY OF HBV**

(71) Applicant: RCMG Inc., Tsukuba-shi, Ibaraki 305-0047 (JP)
(72) Inventor: NARIMATSU, Hisashi, Tsukuba-shi, Ibaraki 305-0047 (JP); ANGATA, Kiyohiko, Tsukuba-shi, Ibaraki 305-0047 (JP); YONEYAMA, Hiroyuki, Tsukuba-shi, Ibaraki 305-0047 (JP)
(74) Representative: Grünecker Patent- und Rechtsanwälte PartG mbB
(86) International application number: PCT/JP2020/021721
(87) International publication number: WO 2021/245776

(57) **Abstract**

The present invention is to provide an anti-RNA virus particle antibody of HBV which specifically identifies RNA virus particle of hepatitis B virus (HBV).

## Description

### TECHNICAL FIELD

The present invention relates to an RNA virus particle (RNA virion) antibody of hepatitis B virus (HBV), a method for detecting RNA virus particles of HBV using the same, and a kit for detecting RNA virus particles of HBV.

### BACKGROUND ART

Hepatitis B virus (HBV), which has about 2 billion already infected persons and about 300 million chronically infected patients around the world, causes chronic hepatitis, hepatic cirrhosis and liver cancer, occupies 20 to 30% of causes of liver cancer, and is an infectious disease that kills 880,000 people annually (Non-Patent Documents 1 to 5). In 1970, the Dane paticle (Dane particle), which is the main body of infection, was discovered, in 1972, it became possible to carry out diagnosis by a qualitative inspection of HBs antigen (Hepatitis B virus surface antigen) which an envelope thereof, thereafter, in 1979, the main body of the Dane particle was found to be a DNA-containing virion (complete virion or DNA virion) by cloning of HBV-DNA, and in 1980, diagnosis of HBV-DNA inspection by the PCR method was widely carried out (Non-Patent Document 6). After that, it has become possible to grasp the infected state in more detail by various inspection methods such as HBe antigen and antibody, etc., HBV-DNA remains the mainstay of monitoring today to determine whether infected or not.

In the field of therapy, interferon alpha (IFN-α), which requires antiviral activity, was introduced in 1990, and Lamivudine, the first nucleic acid analog therapeutic agent, in 1998, and since 2000, pegylated interferon (Peg-IFN) and various nucleic acid analog preparations (NUC) became the mainstay of treatment (Non-Patent Document 6). HBV infection has come to be considered as a controllable infectious disease since hepatitis B virus surface antigen (HBsAg) and HBV-DNA which are HBV markers can be suppressed. However, on the other hand, there has existed a phenomenon of HBV infection by blood transfusion from blood donors with negative existing inspections or by needle-stick accidents. A more serious problem in the case of HBV is that asymptomatic infected persons unknowingly infect healthy subject and become fulminant. A new question was whether existing inspections were sufficient or not. Therefore, in the 2010s, attention was focused on the development of methods to quantify HBsAg (qHBsAg) and to detect HBV-DNA by next-generation PCR methods in order to enhance detection sensitivity.

Under such circumstances, a receptor for HBV to infect hepatocytes was firstly clarified in 2012 (Non-Patent Document 7). Since it became possible to study the life cycle of HBV in hepatocytes became possible, and the *in vitro* experiment system was finally established, and its elucidation was promoted at once. It was found that HBV not only migrates into the nucleus and is integrated into human genes (integrated DNA), but also remains in the liver for a long period of time as completely closed doublestranded DNA (convalently closed circular deoxyribonucleis acis: cccDNA) in the nucleus. This cccDNA is considered to be the essence that causes HBV reactivation. However, in the current IFN and NCU treatment, complete elimination is not possible (Non-Patent Documents 8 to 11).

Although a vaccine against HBV exists, about 30 million people are still newly infected annually and 4.5 million people continue treatment for chronic hepatitis (Non-Patent Documents 1 and 2). There is no doubt that the goal of HBV treatment is to improve life expectancy and QOL, but the specific treatment goals in clinical practice are 1) negative or suppressed HBV-DNA in blood as a short-term goal, and 2) disappearance of qHBsAg in blood as a long-term goal (Non-Patent Documents 9 to 11). According to this, it becomes the state of a clinical cure, and a risk of carcinogenesis is reduced. Since measurement of cccDNA cannot be carried out without an invasive liver biopsy, it is not practically feasible, and it can be considered that by the disappearance of qHBsAg in blood, cccDNA in the liver could be almost completely eliminated. As a current new therapeutic goal, a paradigm shift has occurred from negative or suppression of HBV-DNA in blood) to disappearance of qHBsAg (HBsAg loss) (Non-Patent Documents 9 to 11).

Due to NCU treatment markedly progressed in recent years, it has become possible to achieve Sustained virological resoponse (SVR: sustained virological suppression), and even to acquire negative HBV-DNA in blood relatively quickly. However, "negative" is, to be exact, "suppression below the limit of measurement detection", and a very sensational fact was experimentally established for the first time in 2019 that in fact the serum of patients whose HBV-DNA in blood is negative during NCU treatment has infectious potential (Non-Patent Document 12). After HBV-DNA in blood becomes negative, it usually takes an extremely long period of time before qHBsAg in blood disappears, or in most cases, it does not disappear for a lifetime (Non-Patent Document 13). Since there is no way to measure whether such long-term suppression of HBV-DNA in blood, or whether infectious potential is lost during the period of negative of HBV-DNA in blood or not, it is difficult to determine when NUC can be withdrawn, and patients are forced to take long-term doses for almost a lifetime. Accordingly, side effects associated with long-term dosing and pressure on the medical economy are also emerged as new and serious issues. Nevertheless, even during that long period of time, measurements of HBV-DNA in blood must be performed about once every three months to monitor for signs of reactivation, etc. Due to the high cost of PCR inspection, pressure on the medical economy becomes also a problem. In addition, in many areas and facilities where PCR equipment and resources are limited, there is also the problem that access to measurement of HBV-DNA in blood itself is extremely difficult. Further, no matter how much the sensitivity of detection is improved, it has almost reached the limit, and the problem of false positive and false negative results due to PCR errors has not been completely eliminated.

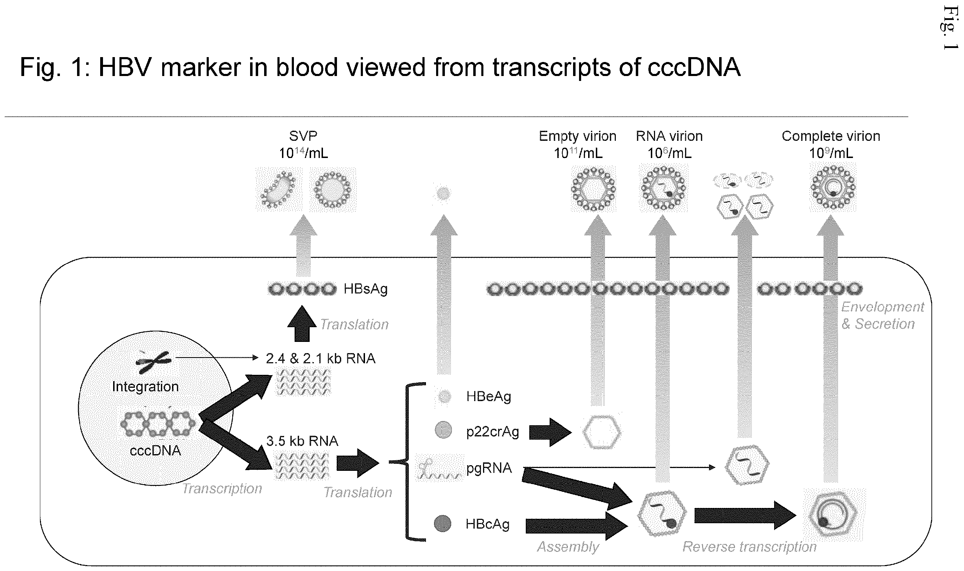

In the need for novel treatments, from the elucidation of cccDNA and the HBV life cycle, development of essential novel therapeutic agent that target the cccDNA itself and its transcriptional activity (Fig. 1) has led to extremely active (Non-Patent Documents 9 to 11). However, in order to verify the effect of these novel agonists, it is a large inhibiting factor that there is currently a lack of indexes that can verify the infectious potential during the extremely long period of time between conventional DNA negativity in blood or qHBsAg disappearance after suppression. Although qHBsAg is a broad index to confirm the presence of HBV infection, 99% or more of them are SVP (subviral particle) having no infectious potential, (which is 100,000-fold or more of DNA particles having actual infectious potential), and it is not possible to efficiently detect DNA paricle having high infectious potential from among them (Non-Patent Document 14). Further, in HBe antigen negative hepatitis which is the main body of long-term SVR cases, it was found that the envelope derived from integrated DNA is more dominant than cccDNA (Non-Patent Document 15). That is, while a definitive index for confirming the presence or absence of HBV is indispensable, it is difficult to grasp the infectious potential over a lifetime, so that another index is desperately needed.

To establish an index to detect HBV infectious potential, in particular, a non-invasive blood marker that does not require biopsy, for a long period of time after HBV-DNA in blood becomes negative or suppressed until qHBsAg in blood disappears is an extremely urgent issue, not only from the perspective of preventing horizontal and vertical infection (transmission) to healthy subject, but also from the perspective of medical costs. In such a background, as an index capable of reflecting transcriptional activity of cccDNA (Fig. 1), HB core-related antigen (HBcrAg) and, since 2018, HBV-RNA are newly attracting attention (Non-Patent Document 13). HBcrAg is a marker based on an excellent theory that detects four core-related antigens that are cccDNA transcripts, that is, HBe antigen, p22r antigen, pregenomic RNA (pgRNA) and HBc antigen, and has attracted attention because of its reported correlation with carcinogenesis (Non-Patent Document 16). However, disadvantages do not overcome that it essentially requires artificial pretreatment of releasing core-related antigens from envelope particles and immune complex and the measurement range is as narrow as 3-7 logU/mL so that in high-value cases exceeding 7, additional dilution is necessary, or in low-value cases below 3 (almost all the cases of DNA negative are correspond to), monitoring reflecting accurate disease status cannot be carried out. (Non-Patent Documents 13 to 14).

In 2016, it was experimentally demonstrated that HBV pgRNA exists in blood as virion (virus particles) coated with an envelope (Non-Patent Document 17). It has suddenly begun to attract attention as a novel marker reflecting cccDNA transcriptional activity and reservoir size, and is now a very hot new research area. There are a series of reports suggesting that it is useful as a predictive marker for HBe antigen seroconversion, a marker for determining NUC discontinuation, and for determining the effect and discontinuation of IFN in HBe antigen negative hepatitis, which more reflects the reservoir size (Non-Patent Documents 18 to 20). Since continuous disease monitoring by cccDNA measurement by liver biopsy is practivally impossible, it has emerged as the most promising blood marker. However, while it is active at the research level, there remains a problem in its practicality as a clinical diagnostic agent. Although the RACE method has been replaced by the PCR method, there is still no consensus on a PCR method and it is mixed at the laboratory level, so future adjustment is a major hurdle (Non-Patent Documents 13 to 14). In addition, even after the PCR method is established, it can be easily predicted that problems such as cost and resource limitation will occur, as with HBV-DNA, and rather, storage conditions of serum are stricter than those of DNA, which is expected to be an added hurdle in this regard.

### PRIOR ART DOCUMENTS

### PATENT DOCUMENT

WO2019/235584 METHOD FOR EFFICIENTLY INDUCING ANTIBODY TO HEPATITIS VIRUS, ANTIBODY AND DETECTION SYSTEM

### NON-PATENT DOCUMENTS

1) WHO. Guidelines for the prevention, care and treatment of persons with chronic hepatitis B infection. March 2015.
2) WHO. Guidelines on hepatitis B and C testing. February 2017.
3) Terrault NA, Loch ASF, McMahon BJ et al. Update on prevention, diagnosis, and treatment of chronic hepatitis B: AASLD 2018 hepatitis B guidance. Hepatol 67: 1560, 2018.
4) European association for the study of the liver. EASL 2017 clinical practice guideline on the management of hepatitis B virus infection. J Hepatol 67: 370, 2017.
5) The Japan Society of Hepatology, Hepatitis Diagnosis Guideline Preparing Committee. Hepatitis B Treatment Guideline (Vol. 3.1). March 2019.
6) Marzio DH, Hann H. Then and now: The progress in hepatitis B treatment over the past 20 years. WJG 20: 401, 2014.
7) Yan H, Zhong G, Xu G et al. Sodium taurocholate cotransporting polypeptide is a functional receptor for human hepatitis B and D virus. eLife 1: e00049, 2012.
8) Nassal M. HBV cccDNA: viral persistence reservoir and key obstacle for a cure of chronic hepatitis B. Gut 64: 1972, 2015.
9) Lok AS, Zoulim F, Dusheiko G et al. Hepatitis B cure: From discovery to regulatory approval. J Hepatol 66: 1296, 2017.
10) Revill PA, Chisari FV, Block jM et al. A global scientific strategy to cure hepatitis B. Lancet Gastroenterol Hepatol. 4: 545, 2019.
11) Cornberg M, Lok AS. Terrault NA et al. Guidance for design and endpoints of clinical trials in chronic hepatitis B - Report from the 2019 EASL-AASLD HBV treatment endpoints conference. J Hepatol 72: 539, 2020.
12) Burdette D. Evidence for the presence of infectious virus in the serum from chronic hepatitis B patients suppressed on nucleos(t)ide therapy with detectable but not quantitative HBV DNA. J Hepatol 70: suppl e95, 2019.
13) Charre C, Levrero M, Zoulin F, et al. Non-invasive biomarkers for chronic hepatitis B virus infection management. Antiviral Res 169: 104553, 2019.
14) Hu J, Liu K. Complete and incomplete hepatitis B virus particles: Formation, function, and application. Viruses 9: 56, 2017.
15) Wooddell CI, Yuen MF, Chan HLY et al. RNAi-based treatment of chronically infected patients and chimpanzees implicates integrated hepatitis B virus DNA as a source of HBsAg. Sci Trans Med 9: 409, 2017.
16) Inoue T, Tanaka Y. The role of hepatitis B core-related antigen. Genes 10: 357, 2019.
17) Wang J, Shen T, Huang X et al. Serum hepatitis B virus RNA is encapsidated pregenome RNA that may be associated with persistence of viral infection and rebound. J Hepatol 65: 700, 2016.
18) Liu S, Zhouu B, Valdes JD et al. Serum HBV RNA: a new potential biomarker for chronic hepatitis B virus infection. Hepatol 69: 1816, 2019.
19) Liu YY, Liang XS. Progression and status of antiviral monitoring in patients with chronic hepatitis B: From HBsAg to HBV RNA. WJH 10: 603, 2018.
20) Farag MS, van Campenhout MJH, Pfefferkorn M et al. Hepatitis B virus RNA as early predictor for response to pegylated interferon alpha in HBeAg-negative chronic hepatitis B. Clin Infect Dis pil: ciaa013, 2020.
21) Wagatsuma T, Kuno A, Angata K et al. Highly sensitive glycan profiling of hepatitis B viral particle and a simple methods for Dane particle enrichment. Anal Chem 90: 10196, 2018.

### SUMMARY OF THE INVENTION

### PROBLEMS TO BE SOLVED BY THE INVENTION

An object of the present invention is to specifically identify RNA virus particles (RNA virion) of hepatitis B virus (HBV).

### MEANS TO SOLVE THE PROBLEMS

HBsAg is constituted by SVP (10¹⁴/mL) having no infectious potential, empty particle (Empty virion: 10¹¹/mL) containing no DNA and RNA, complete virion (Dane particle: 10⁹/mL) containing DNA which is the main body of infectious potential, and virion (RNA virion: 10⁶/mL) containing RNA that has been proven to exist in recent years. However, its existing ratio in blood is estimated that, as shown in the parentheses (), when the RNA virion is made 1, the DNA virion is 1,000-fold, the Empty virion is 100,000-fold, and the SVP is 100,000,000-fold (Non-Patent Document 14). Although qHBsAg and HBcrAg can theoretically react with the RNA virion, the existing ratio of the RNA virion is thus extremely small. Even if the measurement sensitivity of the qHBsAg is increased, it is not the level that can solve the problem.

The envelope of HBsAg is constituted by S protein, M protein and L protein irrespective of whether or not it contains RNA inside thereof. It has been found that even when the proteins have the same constitution, glycosylation patterns thereof are different from each other, and a method for efficiently detecting virion from a huge amount of HBsAg has been searched for (Non-Patent Document 21).

An antibody (hereinafter: referred to as "Hepatitis B surface antigen glycan isomer: HBsAgGi") that recognizes O-glycosylated Pre-S2 protein (O-glycosylated Pre-S2) has been known, and when infection-inhibiting test of HBV virus *in vitro* is carried out, it has been found that it has a neutralizing activity (Patent Document 1).

The present invention is based on the discovery that HBsAgGi specifically recognizes RNA virion. It was never predictable that the possibility to bind to RNA virion in a blood sample in a real living body using HBsAgGi. This is because, in particular, extremely enormous and various sugar chains including sugar chains derived from meals exist in human blood, so that it is generally difficult to detect the target glycoprotein from a blood sample even if an antibody against glycoprotein is used.

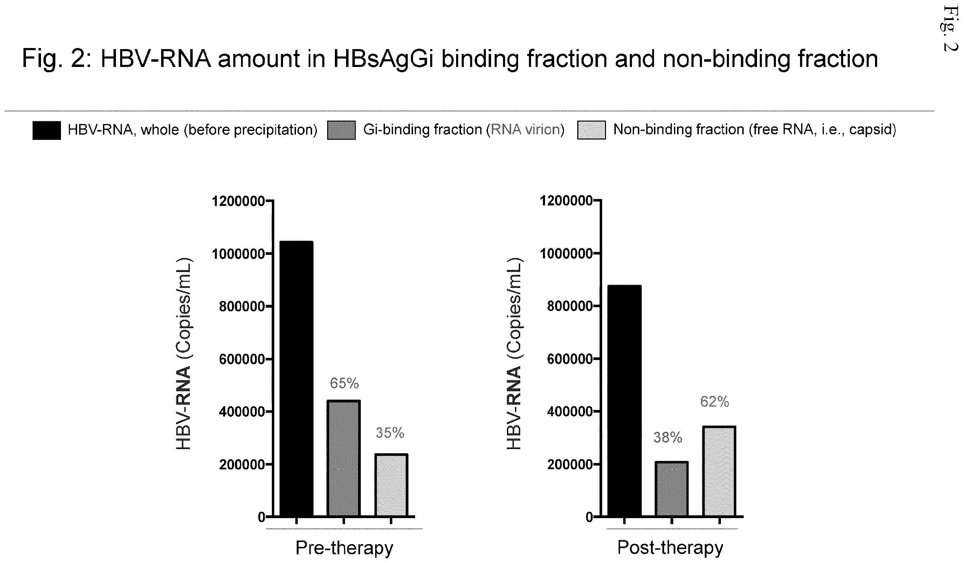

The inventors carried out an immunoprecipitation test with HBsAgGi using actual sera from patients with chronic hepatitis B and diligently investigated what was recognized in the fraction that binds to HBsAgGi, and surprisingly, it was clarified that it recognized HBV-RNA in blood (Fig. 2). This finding meant that it bound to the envelope of virions containing RNA, and it was found that it has a completely unexpected action of exactly capturing RNA virion. And yet, RNA virion in blood could be detected not only in the state of a high value of HBV-DNA before treatment of NUC but also in patients whose HBV-DNA in blood became negative by treatment of NUC.

Further, it was also found that discrimination between RNA virion and free RNA is possible by measuring the HBV-RNA value before immunoprecipitation and the HBV-RNA value in the fraction that does not bind to HBsAgGi (Fig. 2).

In patients whose HBV-DNA in blood becomes negative or SVR by the NCU treatment, during a long period of time of continuing treatment aiming HBsAg loss, HBV-RNA in blood is becoming more important as a marker that can grasp infectious potential and capable of becoming a guide for discontinuing or resuming a medicine, but the problem that a diagnostic method by PCR has not been established and the possible measurement areas and facilities are limited and has not been overcome.

The present application is to provide a method which is capable of measuring HBsAgGi with point-of-care and bedside-level using a molecule that can easily and simply detect and measure RNA virion by an immunological method.

Until now, in the measurement of the amount of viral DNA or RNA, a method of measuring it by amplifying the copy by PCR was the mainstream, but the present invention is an epoch-making technique that changes such a situation at once. Because HBV is a silent epidemic virus in which infected persons are widely present all over the world, PCR inspection alone, which is expensive and requires equipment, is not sufficient to pick up patients having infectious potential, and infections continue to occur. The present invention that enables a simple and rapid inspection will contribute to development in areas where it has not been possible to pick up and to prevention of infection from asymptomatic infected person to healthy subject on a global scale.

More specifically, the present invention is to provide the following [1] to [5].
[1] An anti-RNA virus particle antibody of HBV, which specifically discriminates RNA virus particles of hepatitis B virus (HBV).
[2] The anti-RNA virus particle antibody of HBV described in the above-mentioned [1], which contains CDR sequence in a heavy chain amino acid sequence shown in SEQ ID NO: 1 and CDR sequence in a light chain amino acid sequence shown in SEQ ID NO:2,
   CDR sequence in a heavy chain amino acid sequence shown in SEQ ID NO:3 and CDR sequence in a light chain amino acid sequence shown in SEQ ID NO:4,
   CDR sequence in a heavy chain amino acid sequence shown in SEQ ID NO:5 and CDR sequence in a light chain amino acid sequence shown in SEQ ID NO:6,
   CDR sequence in a heavy chain amino acid sequence shown in SEQ ID NO:7 and CDR sequence in a light chain amino acid sequence shown in SEQ ID NO:8, or
   a heavy chain CDR sequence and a light chain CDR sequence each having 70% identity with these
[3] The anti-RNA virus particle antibody of HBV described in the above-mentioned [2], which contains CDR sequence in the heavy chain amino acid sequence shown in SEQ ID NO: 1 and CDR sequence in the light chain amino acid sequence shown in SEQ ID NO:2.
[4] The anti-RNA virus particle antibody of HBV described in any one of the above-mentioned [1] to [3], which contains the heavy chain shown in SEQ ID NO:1 and the light chain shown in SEQ ID NO:2,
   the heavy chain shown in SEQ ID NO:3 and the light chain shown in SEQ ID NO:4,
   the heavy chain shown in SEQ ID NO:5 and the light chain shown in SEQ ID NO:6,
   the heavy chain shown in SEQ ID NO:7 and the light chain shown in SEQ ID NO:8, or
   heavy chain and light chain each having 70% identity with these.
[5] The anti-RNA virus particle antibody of HBV described in the above-mentioned [4], which contains the heavy chain shown in SEQ ID NO: 1 and the light chain shown in SEQ ID NO:2.

The present invention further relates to the following.
[6] A detection method of RNA virus particles of HBV, which comprises a step of contacting the antibody described in any one of the above-mentioned [1] to [5] with a sample.
[7] The detection method described in the above-mentioned [6], which comprises a step of treating a sample with Dnase.
[8] The detection method described in the above-mentioned [6] or [7], wherein the sample is derived from a patient who is now treating with or has been treated with a nucleic acid analog preparation.
[9] The detection method of RNA virus particles of HBV described in any one of the above-mentioned [6] to [8], wherein the sample is derived from a patient whose suppression of HBV-DNA in blood is confirmed.
[10] A kit for detecting RNA virus particles of HBV, which comprises the antibody described in any one of the above-mentioned [1] to [5].
[11] A method for providing data for planning prevention or treatment of HBV, which comprises a step of contacting the antibody described in any one of the above-mentioned [1] to [5] with a sample.

### EFFECTS OF THE INVENTION

In patients of chronic hepatitis B (CHB), while NUC can now achieve HBV-DNA negative or sustained suppression, which has been the main goal of conventional treatment, it has become clear that there remains infectious capacity in such patient sera, and it comes the situation that conventional inspection alone cannot be used at all. Under such circumstances, 30 million new HBV-infected patients are still appearing every year. Therefore, novel HBV markers are earnestly desired in grasping infectious potential and decision whether to continue or discontinue treatment, and in particular, importance of measuring HBV-RNA in blood is extremely heightening. As the molecular biological methods for detecting HBV-RNA, there exist only the RACE method and the PCR method, and there is no practical diagnostic method and the development is still at the laboratory level.

According to the immunological means of the present invention, that is, the antigen-antibody reaction, it is firstly clarified that RNA virion coated with envelope can be detected quickly and easily among HBV-RNA, and further RNA virion and free RNA (nucleocapsid RNA) can be discriminated.

Conventionally, it has not been considered to capture RNA virion by a method other than PCR, but according to the present invention, it is possible to epochally detect RNA virion by a simple and widely used immunological method.

According to the present invention, judgement of the HBV infectious potential is possible at low cost and in any environment without using the PCR method, which requires expensive reagents and devices and limits the facilities and areas where measurement can be performed, and it is expected to promote QOL of CHB patients and further medical economic effect, and contribute to reduction of newly infected persons by silent endemic virus.

### BRIEF DESCRIPTION OF THE DRAWINGS

Fig. 1 is a drawing showing whether the transcript of cccDNA which is the main body of HBV infection is associated with any material among various kinds of HBV markers in blood. A typical comparison of existing amount of four kinds of virus particles (virion), that is SVP, empty virion, DNA virion and RNA virion, in the blood of CHB patients is shown by the number of copies in 1 mL of blood.
Fig. 2 shows the number of HBV-RNA in the serum of CHB patients before starting NCU treatment, and the number of the same in the Gi-binding fraction and non-binding fraction in which the serum of CHB patients 48 weeks after the NCU treatment where HBV-DNA in blood became negative (suppressed below the detection limit) were subjected to immunoprecipitation by HBsAgGi (Gi).

### EMBODIMENTS TO CARRY OUT THE INVENTION

Hereinafter, the present invention will be explained in detail.

The present inventor has found that by preparing an antibody (HBsAgGi) which recognizes an O-glycosylated Pre-S2 protein (O-glycosylated Pre-S2), and by recognizing HBsAgGi, RNA virion in blood can be detected.

In the present invention, "Hepatitis B" is also expressed as HBV, and may be any of chronic hepatitis B, acute hepatitis B and fulminant hepatitis B, and "hepatitis B virus" means a virus having an ability of developing these hepatitis B. The present invention is to advantageously provide an antibody specifically discriminate RNA virus particles of chronic hepatitis B (CHB).

In the present invention, "RNA virus particle" may be any HBV particle that contains RNA.

The antibody in the present invention may be a polyclonal antibody or a monoclonal antibody as long as it is an anti-RNA virus particle antibody of HBV that specifically discriminate the RNA virus particle antigen of hepatitis B virus. Also, the antibody of the present invention may be any antibody that can be produced by a commonly used method in this technical field of the art.

In particular, the antibody of the present invention is preferably an antibody containing
CDR sequence in the heavy chain amino acid sequence shown in SEQ ID NO:1 and
CDR sequence in the light chain amino acid sequence shown in SEQ ID NO:2,
CDR sequence in the heavy chain amino acid sequence shown in SEQ ID NO:3 and
CDR sequence in the light chain amino acid sequence shown in SEQ ID NO:4,
CDR sequence in the heavy chain amino acid sequence shown in SEQ ID NO:5 and
CDR sequence in the light chain amino acid sequence shown in SEQ ID NO:6,
CDR sequence in the heavy chain amino acid sequence shown in SEQ ID NO:7 and
CDR sequence in the light chain amino acid sequence shown in SEQ ID NO:8, or
CDR sequence containing amino acid sequences having sequence identity of at least 70%, 75%, 80%, 85%, 90%, 95%, 98% or 99% to three heavy chain CDR sequences and light chain CDR sequences in these, and more preferably contains three heavy chain
CDR sequences in the amino acid sequence shown in SEQ ID NO: 1, and three light chain CDR sequences in the amino acid sequence shown in SEQ ID NO:2.

Further, the antibody of the present invention is preferably an antibody containing
the heavy chain shown in SEQ ID NO: 1 and the light chain shown in SEQ ID NO:2,
the heavy chain shown in SEQ ID NO:3 and the light chain shown in SEQ ID NO:4,
the heavy chain shown in SEQ ID NO:5 and the light chain shown in SEQ ID NO:6,
the heavy chain shown in SEQ ID NO:7 and the light chain shown in SEQ ID NO:8, or
heavy chain and light chain containing amino acid sequence having sequence identity of
at least 70%, 75%, 80%, 85%, 90%, 95%, 98% or 99% to these heavy chains and light chains, respectively.

Incidentally, the combination of the heavy chain CDR sequence and light chain CDR sequence may be any combination of the above-mentioned CDR sequences.

The present invention more advantageously relates to an anti-RNA virus particle antibody of HBV which specifically discriminate RNA virus particles of HBV, particularly to an antibody that recognizes O-glycosylated Pre-S2 protein which is specific to RNA virus particles of HBV, and more particularly to an anti-RNA virus particle antibody of HBV containing the heavy chain which contains CDR1 sequence, CDR2 sequence, and CDR3 sequence in the amino acid sequence of the heavy chain shown in SEQ ID NO: 1, and the light chain which contains CDR1 sequence, CDR2 sequence, and CDR3 sequence in the amino acid sequence of the light chain shown in SEQ ID NO:2.

The present invention further advantageously relates to an antibody which contains the heavy chain shown in SEQ ID NO: 1 and the light chain shown in SEQ ID NO:2.

The present invention also relates to a nucleic acid having a base sequence encoding the amino acid sequence of the above-mentioned antibody. Advantageously, the present invention relates to a nucleic acid having, the base sequence shown in SEQ ID NO:9 encoding the amino acid sequence shown in SEQ ID NO: 1, the base sequence shown in SEQ ID NO: 10 encoding the amino acid sequence shown in SEQ ID NO:2, the base sequence shown in SEQ ID NO: 11 encoding the amino acid sequence shown in SEQ ID NO:3, the base sequence shown in SEQ ID NO: 12 encoding the amino acid sequence shown in SEQ ID NO:4, the base sequence shown in SEQ ID NO: 13 encoding the amino acid sequence shown in SEQ ID NO:5, the base sequence shown in SEQ ID NO: 14 encoding the amino acid sequence shown in SEQ ID NO:6, the base sequence shown in SEQ ID NO: 15 encoding the amino acid sequence shown in SEQ ID NO:7, or the base sequence shown in SEQ ID NO: 16 encoding the amino acid sequence shown in SEQ ID NO:8.

The present invention also relates to an expression vector containing the above-mentioned nucleic acid.

The antibody in the present invention can be produced by a general method known in this technical field of the art.

The present invention is to provide a detecting method of RNA virus particles of HBV which comprises a step of contacting the above-mentioned antibody with a sample.

In the detecting method of the present invention, it advantageously comprises a step of treating the sample with Dnase.

The producing method and the detecting method of the above-mentioned antibody of the present invention may be any method commonly used in this technical field of the art. Preparation of HBsAgGi, and the ELISA method and chemiluminescence method using the same can be appropriately carried out by a person skilled in the art with the general immunological inspection method in this technical field of the art. In addition, those skilled in the art can easily adapt the present invention to various measurement instruments. Further, it is also easily possible for those skilled in the art to prepare a simple and rapid test kit by a finger-stick method, etc. In particular, preparation and inspection method of HBsAGgGi of the present invention can be carried out by the description of, for example, WO2019/235584.

The subject of the inspection in the present invention is all individuals infected with HBV. It can be applied to all, not only to patients who continue to be treated with HBV therapeutic agents, but also to patients who have stopped treatment and are being monitored, and asymptomatic carriers.

By grasping the HBV infectious potential, it can be used for whole HBV medical treatment such as judgment of therapeutic effect, selection of therapeutic agents, determination of treatment policy, judgment of continuation or discontinuation of treatment, etc.

In the present invention, as the subject of the inspection, it can be used for healthy subjects having high risk of infecting HBV. Specifically, it includes healthcare workers, families of HBV-infected persons, etc. (all populations listed in WHO guidelines on hepatitis B and C testing 2017).

In the present invention, as the subject of the inspection, it can be used for blood donation donors. It contributes to the prevention of infection at the waterside, etc.

In the present invention, as the subject of the inspection, it can be used for pregnant women. It contributes to the prevention vertical ward infections, etc.

In the present invention, as the subject of the inspection, it can be used for a preoperative inspection of surgery, a preoperative inspection of endoscopic examination, etc. It contributes to judge whether a risk of horizontal infection causes or not by the inspection to be carried out or surgery.

In the present invention, as the subject of the inspection, it can be used for patients presenting with a liver disorder such as fatty liver. By distinguishing patients whose HBV infection overlaps with or is hidden behind liver disorder, it contributes to the revision of treatment policy and the improvement of patient QOL.

The subject of the inspection in the present invention is all adults. In the prior art, it has been reported that only 10% of true HBV-infected persons can be diagnosed with HBV infection (Literature: Polaris Observatory Collborators Global prevalence, treatment, and prevention of hepatitis B virus infection in 2016: a modeling study. Lancet Gastrotenreol Hepatol 3: 383-403, 2018), it is also very meaningful to have an optional inspection for all adults.

The above-mentioned subjects, patients and samples derived from infected person in the present invention may be any biological sample, and there may be mentioned body fluids or tissue extract including blood, serum, saliva, semen, vaginal secretion, and wound exudate of the subject suspected of being infected with hepatitis B virus, and in consideration of ease of sample acquisition and handling, blood, serum and saliva are preferable.

According to the detection method of the present invention, it can advantageously detect RNA virus particles in the samples derived from patients with chronic hepatitis B virus, HBV patients before start, and after the start, after 1 week, 2 weeks, 4 weeks, 8 weeks, 16 weeks, 48 weeks, or after 48 weeks from the start of the nucleic acid analog treatment, patients with confirmed suppression of HBV-DNA in blood, patients in which HBs antigen was disappeared, patients in which HBs antigen is not disappeared in their lifetime, patients before and after the treatment with interferon, or patients before and after administration of novel drugs in the development stage. The sample may be any biological sample. When the sample is derived from patients who have been or had been treated with the nucleic acid analog preparation, it is particularly advantageous.

According to the detection method of the present invention, detection of virus particles in the patients with confirmed suppression of HBV-DNA in blood can be carried out with good accuracy, easily and within a short time, so that it is extremely advantageous.

The patient with confirmed suppression of HBV-DNA in blood in the present invention may be patients who have been confirmed to have reduction of HBV-DNA in blood, patients who have been confirmed to have persistent suppression (SVR) or patients who have been confirmed to have negative HBV-DNA in blood, in the course of treatment of HBV by an arbitrary detection method, for example, PCR method of conventional antibody detection method. In the present specification, HBV-DNA negative (HBV-DNA undetectable) means that the number of detected copies of DNA is 0, and HBV-DNA suppression (HBV-DNA suppression) means that the number of detected copies of DNA is, for example, 4.0 log copies/mL or less, preferably 3.0 log copies/mL or less, and more preferably 2.1 log copies/mL or less. In the present specification, both of HBV-DNA negative and HBV-DNA suppression may be used interchangeably herein.

The present invention relates to a method for providing data for planning prevention or treatment of HBV, which comprises a step of contacting the above-mentioned antibody with a sample. According to the present invention, it is possible to provide data which becomes an index for planning prevention or treatment of HBV, such as recurrence of symptoms and presence or absence of infectivity, which was difficult with the conventional techniques.

The present invention further relates to a method for screening a medicine for prevention or treatment of HBV, which comprises a step of contacting the above-mentioned antibody with a sample. According to the present invention, it is possible to carry out screening of a medicine which becomes a candidate for prevention or treatment of HBV based on more accurate index capable of reflecting transcription activity of cccDNA.

Incidentally, all the prior art references cited in the present specification are incorporated herein in the present specification as a reference.

### EXAMPLES

Hereinafter, the present invention will be explained in more detail by referring to Examples. However, the technical scope of the present invention is not limited to these Examples.

### <Example 1>

Sera of CHB patient were used for immunoprecipitation experiment using HBsAgGi.

### <Antibody used>

HBsAgGi: Antibodies having the heavy chain shown in SEQ ID NO: 1 and the light chain shown in SEQ ID NO:2

### <Patient attribution>

CHB (chronic patient serum):
Chronic hepatitis B (HBs antigen positive persisted for 6 months or longer) patients (n=47) with untreated (no treatment calendar with nucleic acid analog) were as the subject, and sera before treatment with nucleic acid analog (0 week) and 48 weeks after treatment were used.

The numerical value of HBV-DNA in blood before treatment showed a high viral load in blood as 6.6 +/- 1.0 (average +/- SD) Log Copies/mL (n=47).

### <Immunoprecipitation>

In Protein LoBind Tube (Eppendorf) were mixed with a ratio of 2 µL of Biotinylated HBsAgGi (1 µg/µL) and 10 µL of Streptavidin-conjugated magnetic beads and stirred at 4°C for 30 min. To 10 µL of HBsAgGi-magnetic beads were added 10 µL of HBV serum sample and 80 µL of TBS-T and mixed at 4°C.

After 16 hours, the tube was recovered and continued to spin down, and allowed to stand on the magnet stand. The supernatant was transferred to a new tube, and used as a sample.

In addition, in order to make the amount of the total RNA component, which is the sum of RNA virus particles and Free RNA fraction, as the baseline, a PCR detection system was also carried out. In the PCR detection system, HBV RNA was extracted using RNeasy Mini Kit (QIAGEN) (eluted at 40 µL).

After treating 8 µL of RNA extracted from patient serum sample with DNase (Promega), RT reaction was carried out using SuperScript IV (Thermo Fisher) and H-RT (5'-GACGTTGTAAAACGACGGCCAGGCCTCAAGGTCGGTCGTTGAC-3') to synthetize cDNA (as cDNA sample derived from patient serum).

Using a cDNA sample derived from patient serum, PCR reaction was carried out. As the primer in the PCR reaction, H-F (5'- CTGTGCCTTCTCATCTGCCG-3'), M13-F (5'- GACGTTGTAAAACGACGGCCAG-3') was used and carried out, and measurement was carried out using Applied Biosystems 7500 Real-Time PCR system (Thermo Fisher).

The details are as follows.

### Step 1: Preparation of HBV-RNA

HBV RNA was extracted using RNeasy Mini Kit (QIAGEN).
1) The fraction containing HBV was dissolved with 600 µL RLT plus buffer (1% 2-Me).
2) It was transferred to gDNA Eliminator spin column.
3) It was centrifuged by a microcentrifuge at room temperature, 8,000 × g for 30 seconds.
4) 600 µL of 70% ethanol was added to the solution passed through the column and mixed.
5) It was transferred to RNeasy spin column, and centrifuged at room temperature, 8,000 × g for 30 seconds.
6) 700 µL of RW1 buffer was added to the upper layer of the column.
7) It was centrifuged at room temperature, 8,000 × g for 15 seconds.
8) 500 µL of RPE buffer was added to the upper layer of the column.
9) It was centrifuged at room temperature, 8,000 × g for 15 seconds.
10) 500 µL of RPE buffer was added to the upper layer of the column.
11) It was centrifuged at room temperature, 8,000 × g for 2 minutes.
12) 40 µL of RNase-free Water was added to the upper layer of the column.
13) It was centrifuged at room temperature, 8,000 × g for 1 minute to elute HBV RNA.

### Step 2: Measurement of HBV-RNA (RT reaction)

1) To remove DNA, the reaction solution shown in the following table was prepared and reacted at 37°C for 30 minutes.

**[Table 1]**

| Reagent | per one reaction |
|---|---|
| RNA (sample derived from serum) | 8 µL |
| RQ1 RNase-Free DNase 10X Reaction Buffer | 1 µL |
| RQ1 RNase-Free DNase | 1 µL |

2) Thereafter, 1 µL of RQ1 DNase Stop Solution was added and reacted at 65°C for 10 minutes. According to this, DNase was deactivated.
3) The reaction solution shown in the following table was prepared and subjected to a reverse transcription reaction (RT) by reacting at 65°C for 5 minutes.

**[Table 2]**

| Reagent | per one reaction |
|---|---|
| DNase treated RNA (sample derived from serum) | 11 µL |
| 2 µM H-RT primer | 1 µL |
| 10 mM dNTP mix | 1 µL |

4) After the RT reaction, the sample was allowed to stand on ice for 1 minute.
5) The reaction solution shown in the following table was added to the sample of 4).

**[Table 3]**

| Reagent | per one reaction |
|---|---|
| 5x SuperScript IV Buffer | 4 µL |
| 100 mM DTT | 1 µL |
| RNase Inhibitor | 1 µL |
| SuperScript IV | 1 µL |

6) The added sample was reacted at 50°C for 15 minutes, and then heated at 80°C for 10 minutes to stop the reaction. 30 µL of DDW was added thereto to make a total 50 µL of HBV cDNA sample.

### Step 3: Measurement of HBV-RNA (PCR reaction)

7) The qPCR reaction solution shown in the following table was prepared.

**[Table 4]**

| Reagent | | per one reaction |
|---|---|---|
| Fast SYBR Green Master Mix (2×) | | 10 µL |
| | H-F primer (100 µM) | 0.05 µL |
| | M13-F primer (100 µM) | 0.05 µL |

8) To 10 µL of cDNA sample (per 1 well) obtained in 6) was added 10 µL of the above-mentioned qPCR reaction solution, and real-time qPCR (Applied Biosystems 7500 Real-Time PCR system, Thermo Fisher) was carried out under the following conditions, and amplified Ct value and Tm value were measured.

**[Table 5]**

| Stages | Temperature | Time | Number of cycles |
|---|---|---|---|
| Holding Stage | 95.0°C | 20 seconds | 1 cycle |
| Cycling Stage | 95.0°C | 3 seconds | 40 cycles |
| | 60.0°C | 30 seconds | |
| Melt Curve Stage | 95.0°C | 15 seconds | 1 cycle |
| | 60.0°C | 60 seconds | |
| | 95.0°C | 15 seconds | |
| | 60.0°C | 15 seconds | |

The amount of HBV RNA was calculated as the average of the Ct values of 2 wells and the relative numerical value (number of copies) of the Standard curve (cDNA of M-HBsAg). The results are shown in Fig. 2.

In the serum of active CHB patient (HBV-DNA = 6.5 logcopies/mL) before NCU treatment, HBV-RNA in blood = 1,043,842 copies/mL, and HBV-RNA of the HBsAgGi-binding fraction after immunoprecipitation was 440,416 copies/m L (65%) and HBV-RNA of the non-binding fraction was 237,094 copies/mL (35%).

In the serum of CHB patient (HBV-DNA = 0 logcopies/mL) who became DNA negative after 48 weeks of NUC treatment, HBV-RNA in blood = 873,953 copies/mL, and HBV-RNA of the HBsAgGi-binding fraction after immunoprecipitation was 207,817 copies/m L (37.8%), and HBV-RNA of non-binding fraction was 341,766 copies/mL (62.2%).

From the above results, according to the present invention, it can be seen that among HBV virus particles in blood, RNA virus particles of HBV, which exist only 1/100,000,000, can be effectively detected. In particular, before treatment, and 48 hours after administration of nucleic acid analog, which is an important time point for treatment plan, and further, even when DNA in blood is the detection limit or less, according to the present invention, an index for effective prevention or treatment, or for diagnosis of HBV can be obtained by carrying out detection of RNA virus particles of HBV.

### UTILIZABILITY IN INDUSTRY

The HBsAgGi of the present invention was found to detect RNA virion in blood by an immunological method. This is an epoch-making method for HBV infection, for which only a measurement method using a molecular biology method such as PCR has been conventionally devised, and yet, it is possible to distinguish it from free HBV-RNA. From these results, it was suggested that HBsAgGi is useful as a diagnostic agent for HBV infectious potential.

## Claims

1. An anti-RNA virus particle antibody of HBV which specifically discriminates RNA virus particles of hepatitis B virus (HBV).

2. The anti-RNA virus particle antibody of HBV according to Claim 1, which contains
CDR sequence in a heavy chain amino acid sequence shown in SEQ ID NO: 1 and CDR sequence in a light chain amino acid sequence shown in SEQ ID NO:2,
CDR sequence in a heavy chain amino acid sequence shown in SEQ ID NO:3 and CDR sequence in a light chain amino acid sequence shown in SEQ ID NO:4,
CDR sequence in a heavy chain amino acid sequence shown in SEQ ID NO:5 and CDR sequence in a light chain amino acid sequence shown in SEQ ID NO:6,
CDR sequence in a heavy chain amino acid sequence shown in SEQ ID NO:7 and CDR sequence in a light chain amino acid sequence shown in SEQ ID NO:8, or
a heavy chain CDR sequence and a light chain CDR sequence each having 70% identity with these.

3. The anti-RNA virus particle antibody of HBV according to Claim 2, which contains
CDR sequence in the heavy chain amino acid sequence shown in SEQ ID NO: 1 and
CDR sequence in the light chain amino acid sequence shown in SEQ ID NO:2.

4. The anti-RNA virus particle antibody of HBV according to any one of Claims 1 to 3, which contains the heavy chain shown in SEQ ID NO: 1 and the light chain shown in SEQ ID NO:2,
the heavy chain shown in SEQ ID NO:3 and the light chain shown in SEQ ID NO:4,
the heavy chain shown in SEQ ID NO:5 and the light chain shown in SEQ ID NO:6,
the heavy chain shown in SEQ ID NO:7 and the light chain shown in SEQ ID NO:8, or
the heavy chain and the light chain each having 70% identity with these.

5. The anti-RNA virus particle antibody of HBV according to Claim 4, which contains the heavy chain shown in SEQ ID NO: 1 and the light chain shown in SEQ ID NO:2.

6. A detection method of RNA virus particles of HBV, which comprises a step of contacting the antibody according to any one of Claims 1 to 5 with a sample.

7. The detection method according to Claim 6, which comprises a step of treating a sample with Dnase.

8. The detection method according to Claim 6 or 7, wherein the sample is derived from a patient who is now treating with or has been treated with a nucleic acid analog preparation.

9. The detection method of RNA virus particles of HBV according to any one of Claims 6 to 8, wherein the sample is derived from a patient whose suppression of HBV-DNA in blood is confirmed.

10. A kit for detecting RNA virus particles of HBV, which comprises the antibody according to any one of Claims 1 to 5.

11. A method for providing data for planning prevention or treatment of HBV, which comprises a step of contacting the antibody according to any one of Claims 1 to 5 with a sample.

12. A method for screening a medicine for prevention or treatment of HBV, which comprises a step of contacting the antibody according to any one of Claims 1 to 5 with a sample.
